# EUROPEAN PATENT APPLICATION

(11) **EP 4 071 767 A1**
(43) Date of publication of application: **12.10.2022**
(21) Application number: 20911402.4
(22) Date of filing: 28.12.2020
(51) Int. Cl.: G16H 50/20, A61B 5/00

(54) **PREDICTION DISPLAY SYSTEM AND TREATMENT METHOD**

(30) Priority: 09.01.2020 US 202062958777 P
(71) Applicant: TERUMO Kabushiki Kaisha, Tokyo 151-0072 (JP)
(72) Inventor: SAWADA Satoshi, Ashigarakami-gun Kanagawa 259-0151 (JP)
(74) Representative: Casalonga
(86) International application number: PCT/JP2020/049238
(87) International publication number: WO 2021/140997

(57) **Abstract**

[Object]

To provide a prediction display system and the like that utilize information obtained before an operation and during the operation, and predict an onset risk of a postoperative complication such as an acute kidney injury which has become a particularly great clinical problem.

[Solution]

A prediction display system is configured such that an onset risk of a complication occurring after an operation is predicted using at least one of pH (hydrogen ion exponent), pO2 (oxygen partial pressure), Ht (hematocrit value), Hb (hemoglobin), DO2 (transportation amount of oxygen), and pCO2 (arterial blood carbon dioxide partial pressure) as parameters of blood gas taken before the operation and during the operation, and the onset risk of the complication occurring after the operation is displayed.

## Description

### Technical Field

The present invention relates to a prediction display system and a treatment method that predict an onset risk of a postoperative complication or the like such as an acute kidney injury.

### Background Art

Conventionally, it has been known that cardiac surgeries or the like accompany postoperative complications such as an acute kidney injury (AKI). These have been great medical problems also from viewpoints of medical economy and patient QOL (Quality Of Life).

Accordingly, in order to predict occurrence of such a postoperative complication in advance, in the case of the AKI, for example, a plurality of biomarkers that are sensitive to a kidney injury and enable detection at an earlier stage than serum creatinine have recently been reported (for example, Patent Document 1 and the like).

In Japan, NGAL (Neutrophil Gelatinase-Associated Lipocalin) and L-FABP (Liver-type Fatty Acid-Binding Protein) are listed in insurance, and globally, there are many reports on NGAL and IGFBP-7/TIMP-2 (Insulin-like Growth Factor Binding Protein-7/Tissue Inhibitor of Metalloproteinases-2). Both can detect nephropathy in a few hours to approximately 12 hours.

### Prior Art Document

### Patent Document

Patent Document 1: Japanese Patent No. 4879993

### Summary of Invention

### Technical Problems

However, a difficulty remains in selecting patients to which the biomarkers and the like are applied, and the biomarkers and the like are relatively expensive. Also, how to change a procedure when a postoperative complication such as an AKI is found has not been established.

In addition, approximately one third of surgical patients develop a postoperative complication such as an AKI. Once a postoperative complication such as an AKI is developed, medical cost, the number of stay days, and the like are greatly increased, and a risk of a chronic renal failure leading to dialysis is reported to increase to nine times. How to prevent the postoperative complication in a risk stage is very important.

On the other hand, though a guideline defining the AKI has made transitions to RIFLE (Risk/Injury/Failure/Loss/End-stage), AKIN (Acute Kidney Injury Network), and KDIGO (Kidney Disease: Improving Global Outcomes), each is defined by changes in urinary volume and serum creatinine (sCr) in a specific period.

However, changes in urinary volume such as oliguria do not occur along with a kidney injury in all cases. In addition, there is a time lag such that serum creatinine increases one to two days after the kidney is damaged. Thus, a preventive intervention is difficult after an increase in serum creatinine is found.

Accordingly, it is an object of the present invention to provide a prediction display system and a treatment method that utilize information obtained before an operation and during the operation, and predict the onset risk of a postoperative complication such as an acute kidney injury which has become a particularly great clinical problem.

### Technical Solution

According to the present invention, the object is achieved by a prediction display system in which an onset risk of a complication occurring after an operation is predicted using at least one of pH (hydrogen ion exponent), pO2 (oxygen partial pressure), Ht (hematocrit value), Hb (hemoglobin), DO2 (transportation amount of oxygen), and pCO2 (arterial blood carbon dioxide partial pressure) as parameters of blood gas taken before the operation and during the operation, and the onset risk of the complication occurring after the operation is displayed.

Preferably, in the prediction display system, the onset risk of the complication after the operation is predicted using at least one of pH, pO2, Ht, Hb, DO2, and pCO2 as the parameters of the blood gas in data obtained during operation of an extracorporeal circulation apparatus and at least one of a pump flow rate, a pump rotational speed, a temperature, a blood circulation time, and an aorta interruption time as operating conditions of the extracorporeal circulation apparatus, and the onset risk of the complication after the operation is displayed.

Preferably, in the prediction display system, the complication is an acute kidney injury occurring after the operation.

Preferably, in the prediction display system, the onset risk of the acute kidney injury after the operation is predicted using at least one of an arterial pressure, a heart rate, an ECG (electrocardiogram), a urinary volume, a peripheral oxygen saturation, and a deep body temperature as a temperature within a body as vital data, and the onset risk of the acute kidney injury after the operation is displayed.

Preferably, in the prediction display system, the onset risk of the complication is an onset risk of at least one of a low cardiac output syndrome (LOS), an acute kidney injury (AKI), a cerebral infarction, a mediastinitis, and an acute respiratory distress syndrome (ARDS) as the complication after the operation.

Preferably, in the prediction display system, a prediction is performed using the onset risk, and an extracorporeal circulation condition of the extracorporeal circulation apparatus, the extracorporeal circulation condition being likely to reduce the onset risk of the complication after the operation, is analyzed and displayed.

Preferably, in the prediction display system, a change in flow rate and rotational speed of either a blood removing pump or a blood sending pump of the extracorporeal circulation apparatus is suggested.

Preferably, in the prediction display system, whether or not there is a need for a hemoconcentrator circuit of the extracorporeal circulation apparatus is determined and displayed on the basis of the onset risk of the complication after the operation.

Preferably, in the prediction display system, the extracorporeal circulation condition is automatically changed on the basis of the prediction.

Preferably, in the prediction display system, an administration system for reducing the onset risk of the complication after the operation is controlled on the basis of the prediction.

Preferably, in the prediction display system, the onset risk of the complication is predicted using at least one of an age, a sex, an address, a race, a family structure, an anamnesis, and a preoperative test value that affect a prognosis as characteristics of a patient, and the onset risk of the complication is displayed.

Preferably, in the prediction display system, the onset risk of the complication is predicted using at least one of an operative method, a lesion site, an operation time, an operator, an anesthesiologist, a dosage, a kind of medicine, an anesthesia induction time, and presence or absence of a blood transfusion as information regarding the operation, and the onset risk of the complication is displayed.

According to the present invention, the object is achieved by a prediction display system in which an index of at least one of the number of ICU (intensive care unit) stay days, presence or absence of in-hospital death, and presence or absence of continuous renal replacement therapy (CRRT) introduction related to medical economy is predicted using at least one of pH, pO2, Ht, Hb, DO2, and pCO2 as parameters of blood gas in data obtained during operation of an extracorporeal circulation apparatus and at least one of a pump flow rate, a pump rotational speed, a temperature, a blood circulation time, and an aorta interruption time as operating conditions of the extracorporeal circulation apparatus, and the index is displayed.

Preferably, in the prediction display system, an onset risk of a main complication and another management item are taken on an axis of ordinate and an axis of abscissa, and are two-dimensionally displayed on a matrix table, and a region to which a transition is made in the matrix table during an ICU stay is predicted and displayed.

Preferably, the matrix table of the prediction display system two-dimensionally displays a group of high onset risks of the main complication and a group of low onset risks of the main complication, and a group of high probabilities of the number of ICU stay days, in-hospital death, and CRRT introduction related to medical economy and a group of low probabilities of the number of ICU stay days, in-hospital death, and CRRT introduction related to medical economy, and the region to which a transition is made in the matrix table during the ICU stay is predicted and displayed.

Preferably, in the prediction display system, the matrix table has a group of high onset risks of the main complication and a group of low onset risks of the main complication, and a group of high risks of occurrence of a symptom that needs an action such as an edema or an infection and a group of low risks of occurrence of the symptom, and the region to which a transition is made two-dimensionally in the matrix table is predicted and displayed.

According to the present invention, the object is achieved by a treatment method including: predicting an onset risk of a complication using at least one of pH, pO2, Ht, Hb, DO2, and pCO2 as parameters of blood gas in data obtained during operation of an extracorporeal circulation apparatus and at least one of a pump flow rate, a pump rotational speed, a temperature, a blood circulation time, and an aorta interruption time as operating conditions of the extracorporeal circulation apparatus; displaying the predicted onset risk of the complication on a screen; and optimizing a circulation condition of the extracorporeal circulation apparatus on the basis of predictive information on the onset risk of the complication, the predictive information being displayed on the screen.

### Advantageous Effect

The present invention has an advantage of being able to provide a prediction display system and a treatment method that utilize information obtained before an operation and during the operation, and predict the onset risk of a postoperative complication such as an acute kidney injury which has become a particularly great clinical problem.

### Brief Description of Drawings

[FIG. 1]

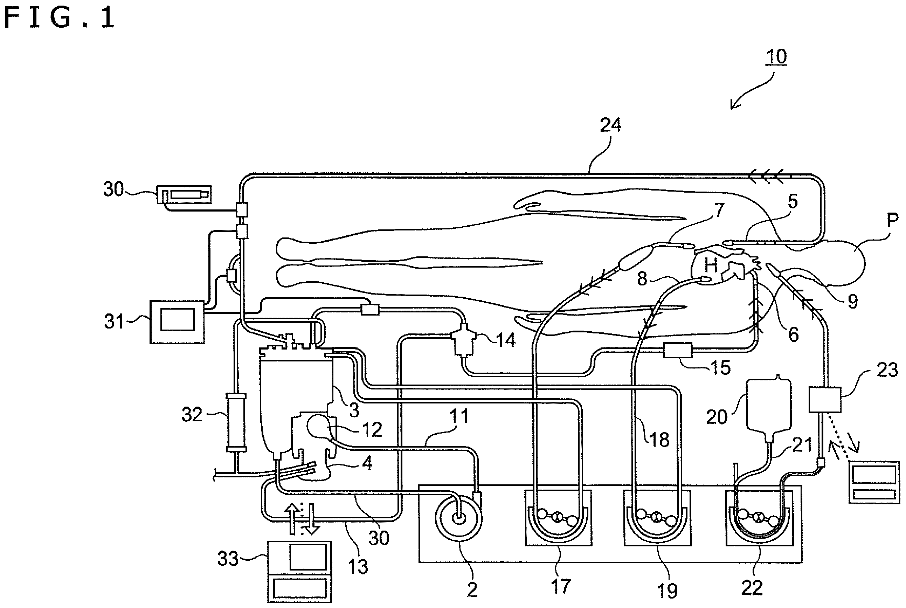

   FIG. 1 is a schematic explanatory diagram illustrating a main configuration of an "extracorporeal circulation apparatus 10" used in a "prediction display system" as an embodiment of the present invention.
[FIG. 2]

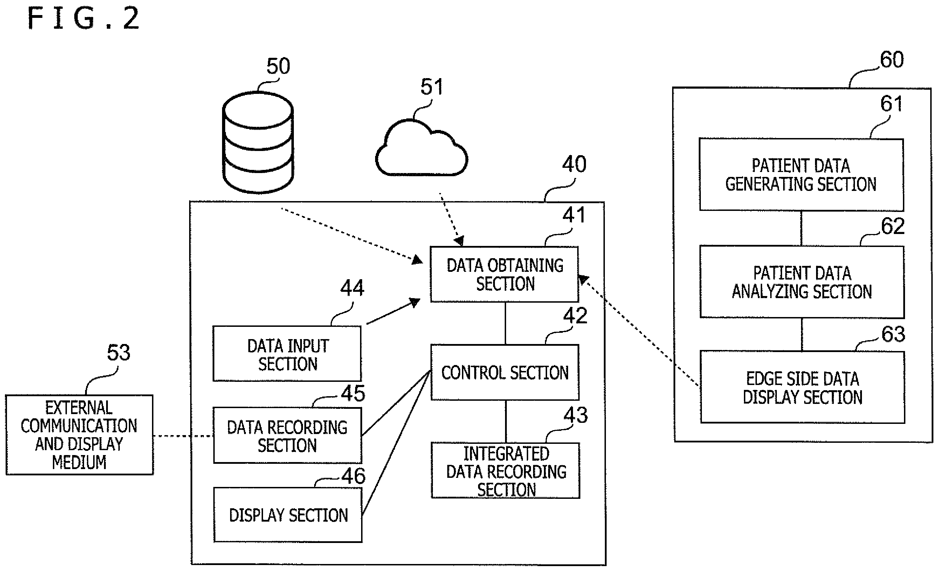

   FIG. 2 is a schematic block diagram illustrating a main configuration of a "controller 40" in FIG. 1 and the like.
[FIG. 3]

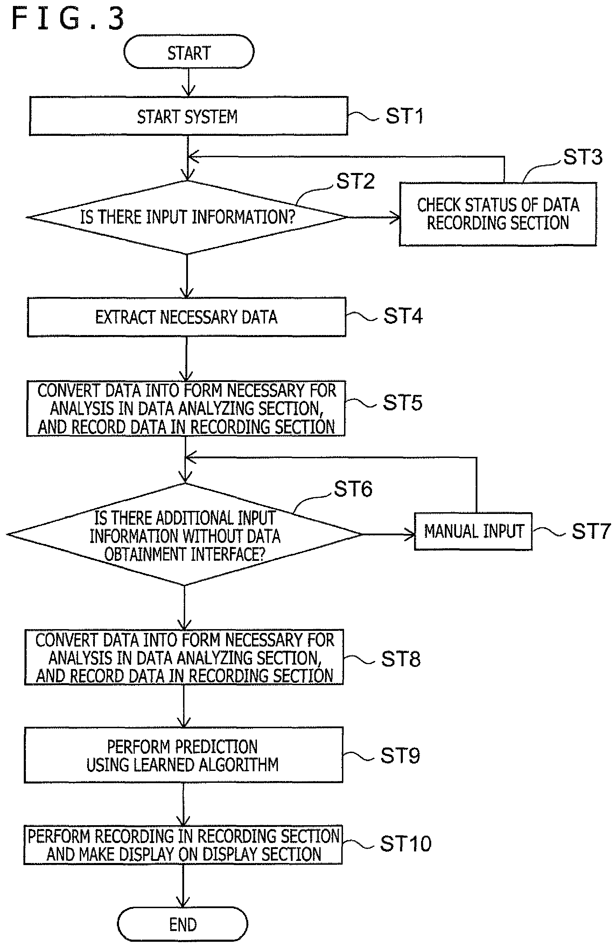

   FIG. 3 is a schematic flowchart illustrating an example of operation of the prediction display system according to the present embodiment and the like.
[FIG. 4]

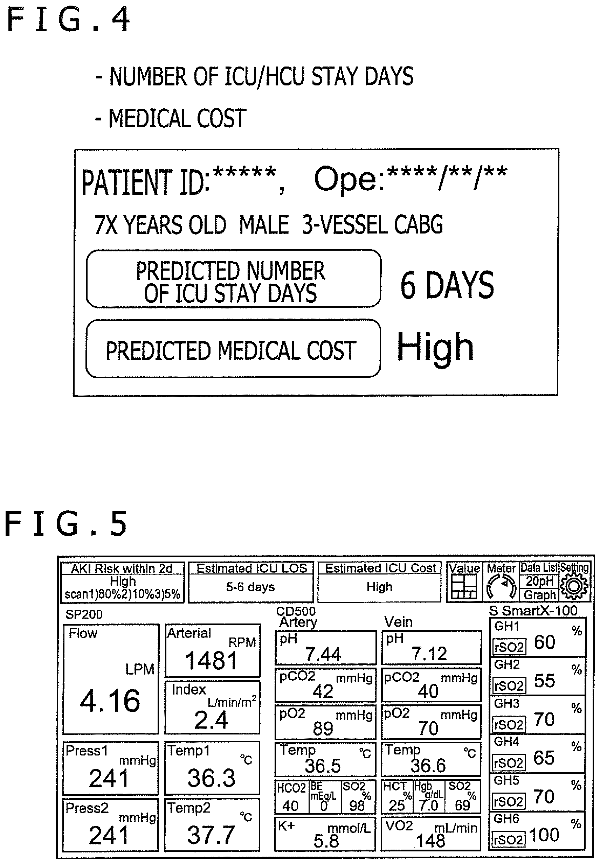

   FIG. 4 is an example of calculating a "medical cost" as an example of medical economy.
[FIG. 5]
   FIG. 5 is a diagram illustrating data as a basis of the "medical cost."
[FIG. 6]

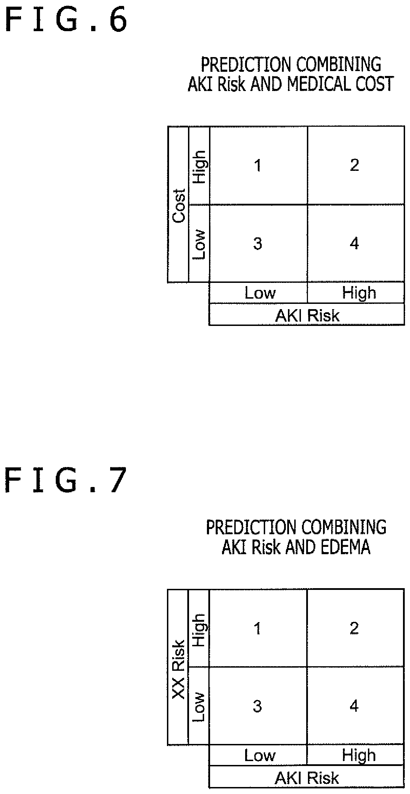

   FIG. 6 illustrates a "matrix table" of an "AKI onset risk" as an example of the onset risk of a postoperative complication and a "medical cost (medical economy or the like)" as an example of medical economy or the like.
[FIG. 7]
   FIG. 7 illustrates a "matrix table" of the "AKI onset risk" and an "edema."

### Modes for Carrying Out the Invention

Preferred embodiments of the present invention will hereinafter be described in detail with reference to the accompanying drawings and the like.

It is to be noted that while embodiments to be described in the following are preferred specific examples of the present invention, and therefore technically preferable various limitations are attached thereto, the scope of the present invention is not limited to these aspects unless the present invention is particularly limited in the following description.

### (First Embodiment)

### (Extracorporeal Circulation Apparatus 10)

FIG. 1 is a schematic explanatory diagram illustrating a main configuration of an "extracorporeal circulation apparatus 10" used in a "prediction display system" as an embodiment of the present invention.

The extracorporeal circulation apparatus 10 illustrated in FIG. 1 performs "extracorporeal circulation operation." In the "extracorporeal circulation operation," the extracorporeal circulation apparatus 10 performs blood circulation operation and gas exchange operation (oxygen addition and/or carbon dioxide removal) for blood in a case where blood circulation in a heart is temporarily stopped by a cardiac surgery, for example.

In a case where a surgery on a heart H of a patient P is performed, for example, the extracorporeal circulation apparatus 10 illustrated in FIG. 1 actuates a blood sending pump 2 as a centrifugal pump of the extracorporeal circulation apparatus 10, and thereby removes blood from the patient P and guides the removed blood to a reservoir 3.

Then, the blood retained in the reservoir 3 is sent to an oxygenator 4, for example, as an oxygenator unit. Thereafter, the blood is oxygenated by performing a gas exchange in the blood by the oxygenator 4. The oxygenated blood is then returned to the patient P. An oxygenator extracorporeal blood circulation can be thereby performed.

Thus, the extracorporeal circulation apparatus 10 is a device that substitutes for the heart and lungs.

As illustrated in FIG. 1, a blood removing cannula 5, a blood sending cannula 6, a suction cannula 7, a vent cannula 8, and a cardioplegia cannula 9 are arranged in the patient P.

In the following, description will be made in order of a circulation circuit from the blood removing cannula 5 to the blood sending cannula 6, a circuit from the suction cannula 7 to the reservoir 3, a circuit from the vent cannula 8 to the reservoir 3, a circuit from a cardioplegic solution bag 20 to the cardioplegia cannula 9, and the like.

### (Circulation Circuit from Blood Removing Cannula 5 to Blood Sending Cannula 6)

As illustrated in FIG. 1, the blood removed via the blood removing cannula 5 inserted and placed in a superior vena cava and an inferior vena cava of the patient P is guided to the reservoir 3 through a blood removing tube 24.

In addition, as illustrated in FIG. 1, an oxygen saturation monitor 30 and a blood gas monitor 31 are formed on the blood removing tube 24.

The blood temporarily retained in the reservoir 3 is guided to the blood sending pump 2 via a first connecting tube 30 in FIG. 1, and is further guided to the oxygenator 4 via a second connecting tube 11, a heat exchanger 12, and the like.

As described above, the blood guided to the oxygenator 4 is oxygenated by performing a gas exchange in the blood by the oxygenator 4, and is thereafter returned to inside of the body of the patient P from the blood sending cannula 6 via a solution feeding tube 13.

Arranged on the solution feeding tube 13 are an artery filter 14 for removing air bubbles in the solution feeding tube 13, an air bubble detector 15 for detecting air bubbles in the blood, and the like.

The reservoir 3 disposed on this circulation circuit is a container that internally has a storage space (storage chamber). The reservoir 3 is configured to temporarily retain the blood removed from the blood removing cannula 5 and the like, the blood emitted during an operation and guided from the suction cannula 7 as will be described later, the blood from the vent cannula 8 extracting air from a left ventricle, an aortic root, or the like as will be described later, and the like.

Because the reservoir 3 thus temporarily retains the blood, the blood removed from the patient P is returned to the patient P without excess or insufficiency.

In addition, the blood sending pump 2 of the circulation circuit is driven by a motor not illustrated or the like. When the blood sending pump 2 is driven, the blood is sent from the blood removing cannula 5 to the blood sending cannula 6 via the reservoir 3, the oxygenator 4, and the like.

In addition, the oxygenator 4 of the circulation circuit performs a gas exchange (oxygen addition and/or carbon dioxide removal) for the blood, as described above. The oxygenator 4 is, for example, a membrane oxygenator, and is preferably a hollow fiber membrane oxygenator. The oxygenator 4 is supplied with an oxygen gas from an oxygen gas supply unit not illustrated.

### (Circuit from Suction Cannula 7 to Reservoir 3)

The blood emitted during the operation is guided from the suction cannula 7 illustrated in FIG. 1 to the reservoir 3 via a suction cannula tube 16, and is retained in the reservoir 3.

A suction pump (blood removing pump) 17 is disposed on the circuit. The blood is guided to the reservoir 3 by an operation of the suction pump 17.

### (Circuit from Vent Cannula 8 to Reservoir 3)

The vent cannula 8 for extracting air from the left ventricle, the aortic root, and the like is guided to the reservoir 3 via a vent cannula tube 18.

A vent pump 19 is disposed on this circuit. The air, the blood, and the like are guided to the reservoir 3 by an operation of the vent pump 19.

### (Circuit from Cardioplegic Solution Bag 20 to Cardioplegia Cannula 9)

A cardioplegic solution is stored in the cardioplegic solution bag 20, and is administered into the patient P via a cardioplegic solution tube 21 and the cardioplegia cannula 9.

A cardioplegic solution pump 22 is disposed on this circuit. The cardioplegic solution is sent to the patient P side by an operation of the cardioplegic solution pump 22.

A heat exchanger 23 is disposed on the cardioplegic solution tube 21.

In addition, as illustrated in FIG. 1, the extracorporeal circulation apparatus 10 has a hemoconcentrator 32, a water cooling and heating controller 33, and the like, and also includes a "controller 40" illustrated in FIG. 2 for performing control of the whole of the extracorporeal circulation apparatus 10 and the like.

FIG. 2 is a schematic block diagram illustrating a main configuration of the "controller 40" in FIG. 1 and the like.

As illustrated in FIG. 2, the controller 40 includes a "data obtaining section 41."

As illustrated in FIG. 2, the data obtaining section 41 obtains various kinds of data from an "EHR (Electronic Health Record) server 50" possessed by a hospital or the like and a cloud 51.

The "EHR server 50" is an electronic health record server. Medical data of patients and the like is recorded on the EHR server 50.

In addition, the EHR server may be a data warehouse (DWH).

As illustrated in FIG. 2, the controller 40 includes a control section 42, and is configured to control the data obtaining section 41, an integrated data recording section 43, a data input section 44, a data recording section 45, a display section 46, and the like.

In addition, as illustrated in FIG. 2, the data recording section 45 of the controller 40 may display contents recorded therein on not only the display section 46 but also an external communication and display medium 53 or the like.

The external communication and display medium 53 includes, for example, the oxygen saturation monitor 30 and the blood gas monitor 31 in FIG. 1 or the like.

Communication may be performed not only in a wired manner but also in a wireless manner.

Further, the data obtaining section 41 of the controller 40 may obtain patient data or the like from a "vital data generating device 60" illustrated in FIG. 2.

As illustrated in FIG. 2, the vital data generating device 60 includes a patient data generating section 61, a patient data analyzing section 62, and an edge side data display section 63.

Incidentally, various kinds of devices such as the controller 40 illustrated in FIG. 1 and FIG. 2, for example, include a computer or the like, that is, include a CPU (Central Processing Unit), a RAM (Random Access Memory), a ROM (Read Only Memory), and the like not illustrated, which are connected to each other via a bus.

When data that needs a certain processing speed is handled, a device having provision therefor is preferable, and a GPU (Graphics Processing Unit), a KPU (Kernel Processing Unit), or the like, for example, may be included.

### (Prediction Timing of Prediction Display System and the Like)

A prediction of the prediction display system according to the present invention is possible in any timing before the operation, during the operation, and after the operation.

Specifically, cases before the operation include not only a case where a prediction is triggered by timing of input of various kinds of data such as vital data of the patient P but also a case where a prediction is performed when data input is completed and a prediction button of the controller 40 or the like is pressed.

In addition, cases during the operation include a case where a prediction is performed in real time according to the vital data of the patient which vital data changes in real time, a case where a prediction is performed at intervals of a certain unit time moving average, a case where a prediction is triggered by timing of data input, and the like.

Then, cases after the operation include, for example, a prediction after processing of data of the patient before the operation and during the operation or the like into a format that can be input into a "learned algorithm."

### (Example of Operation of Prediction Display System according to Present Embodiment)

FIG. 3 is a schematic flowchart illustrating an example of operation of the prediction display system according to the present embodiment and the like.

The flowchart represents an example of making a predictive display of a postoperative complication using preoperative and intraoperative data during the operation on the patient P. Operation and the like will be described in the following by using such an example.

In the present embodiment, the "postoperative complication" includes an acute kidney injury (AKI), a low cardiac output syndrome (LOS), a cerebral infarction, a mediastinitis, an acute respiratory distress syndrome (ARDS), and the like. In the case of the AKI, an onset probability in each AKI stage and an onset probability of the AKI after entry into an ICU are predicted.

First, the extracorporeal circulation apparatus 10 having the present system is used when the patient P undergoes an operation on the heart, for example.

Then, a person in charge of medical information such as a doctor uses the present system to predict a complication such as an AKI after the operation.

First, the system is started in step (hereinafter referred to as "ST") 1 in FIG. 3.

Next, the processing proceeds to ST2. In ST2, whether or not information is input to the controller 40 is determined.

When no information is input in ST2, the processing proceeds to ST3.

In ST3, a status of the "data recording section 45" of the controller 40 in FIG. 2 is checked.

When information is input in ST2, on the other hand, the processing proceeds to ST4.

In ST4, data necessary for a prediction of a "postoperative complication" is extracted from the "data recording section 45" of the controller 40.

Here, the necessary data is, specifically, "preoperative data" and "intraoperative data."

Accordingly, the "preoperative data" and the "intraoperative data" will be described in the following.

### (Preoperative Data)

The preoperative data includes "patient background," "preoperative kidney functions," a "blood test result," "presence or absence of diabetes," "anamnesis information," and the like recorded in the EHR server 50 or the like.

In addition, the preoperative data includes an operative method, a lesion site, an operator, and an anesthesiologist as information regarding the operation.

### (Intraoperative Data)

The intraoperative data is data obtained mainly from the "extracorporeal circulation apparatus 10." The intraoperative data, for example, includes "vital information," "operation information," a "peripheral tissue enzyme level," "blood circulation state information," "heart-lung machine (CPB) circulation state information of the extracorporeal circulation apparatus," and the like.

Each piece of information will be described in the following by citing a specific example.

The "vital information" includes BP (blood pressure), HR (heart rate), ECG (electrocardiogram), a urinary volume, SpO2 (oxygen saturation), an arterial pressure, a peripheral oxygen saturation, a deep body temperature, and the like.

The "operation information" includes an operation time, a CPB (heart-lung machine) driving time, presence or absence of aorta interruption, a dosage, a kind of medicine, an anesthesia induction time, presence or absence of a blood transfusion, and the like.

The "peripheral tissue oxygen level" includes rSO2 (tissue oxygen saturation), SpO2 (oxygen saturation), a pulse rate, and the like.

The "blood circulation state information" includes pH (hydrogen ion exponent), pO2 (oxygen partial pressure), Ht (hydrogen ion exponent), Hb (hemoglobin), DO2 (oxygen delivery), pCO2 (carbon dioxide partial pressure), Temp (temperature), and the like.

The "heart-lung machine (CPB) circulation state information of the extracorporeal circulation apparatus" includes Temp (temperature), a pump rotational speed, a pump flow rate, an aorta interruption time, a blood circulation time, and the like.

In addition, these pieces of data may be used as it is as raw data. Alternatively, a time change graph of all of these parameters may be displayed on the edge side data display section 63 of the vital data generating device 60 in FIG. 2 or the like, a screen thereof may be obtained, and the screen may be set as an input factor. Further, an input factor corresponding to a complication may be set in advance.

Next, the processing proceeds to ST5. In ST5, the data is converted into a form necessary for analysis in the data analyzing section, and is recorded in the data recording section 45.

Next, the processing proceeds to ST6, where whether or not there is additional input information without a data obtainment interface is determined.

When it is determined in ST6 that there is additional input information, the processing proceeds to ST7.

In ST7, necessary data is input manually.

Next, in ST8, the data is converted into a form necessary for analysis in the data analyzing section, and is recorded in the data recording section 45.

Next, the processing proceeds to ST9. In ST9, a prediction is performed by using a learned algorithm.

"Machine learning" is performed in the step in which a "prediction is performed by using the learned algorithm."

The "machine learning" is classified as "supervised learning." The "machine learning" is performed in two separate stages of "learning" and "recognition and prediction."

In the "learning," a "data set" is created, a rule and a pattern of the data are learned on the basis of the data set, and a "learned algorithm" is created.

Then, the "recognition and prediction" of the "machine learning" are performed on the basis of the "learned algorithm."

Next, the processing proceeds to ST10. In ST10, a prediction result is recorded in the data recording section 45, and is displayed on the display section 46.

As a result of the above, predictive information on the onset risk of a postoperative complication is notified to a medical worker such as the doctor during the operation.

### (Predictive Display of Onset Risk of Complication)

"Predictive display of the onset risk of a complication" in the present embodiment includes, for example, predicting a set value that reduces the onset risk of a complication, and displaying the value on the display section 46.

In addition, in the present invention, the onset risk of the complication in a case where the set value is changed to a certain specified value may be displayed at the same time.

More specifically, when information indicating a decrease in BP (blood pressure) as vital information is obtained during the operation, a prediction that a "cerebral infarction risk" increases when the BP (blood pressure) is the same after two minutes is made by the learned algorithm on the basis of a set value, a specified value, or the like, and the prediction is displayed.

In addition, a "start of vasopressor administration" or the like is also displayed as a medical action for improvement.

In addition, as another example, when information indicating a decrease in Ht (hematocrit value) is obtained during the operation, a prediction that "an AKI risk is decreased when water content is reduced" is made by the learned algorithm, and the prediction is displayed.

In addition, "opening of a hemoconcentrator circuit," "preparation of a diuretic," or the like is also displayed as a medical action for improvement.

In addition, in the present embodiment, the onset risk of a complication may be predicted using at least one of an age, a sex, an address, a race, a family structure, an anamnesis, and a preoperative test value that affect a prognosis as characteristics of the patient, and the onset risk of the complication may be displayed.

Further, the onset risk of a complication may be predicted using at least one of an operative method, a lesion site, an operation time, an operator, an anesthesiologist, a dosage, a kind of medicine, an anesthesia induction time, presence or absence of a blood transfusion, and the like as information regarding the operation, and the onset risk of the complication may be displayed.

### (Modification of First Embodiment)

Much of a configuration according to a present modification is similar to that of the foregoing first embodiment, and therefore description thereof will be omitted by providing the same reference symbols or the like. In the following, description will be made mainly of differences.

The foregoing first embodiment is confined to predicting and displaying the onset risk of a postoperative complication or the like and further displaying a medical action for improvement at the same time. However, the present modification further automatically sets and changes a circulation condition (operating condition) of the extracorporeal circulation apparatus 10 on the basis of the prediction of the onset risk of the postoperative complication or the like.

For example, a pump and an analyzing unit are linked to each other, and cooperation with an infusion pump or a syringe pump, feedback on administration, and the like are performed.

In addition, the flow rate and rotational speed of one of the suction pump (blood removing pump) 17 and the blood sending pump 2 of the extracorporeal circulation apparatus 10 may be changed, and the presence or absence of the hemoconcentrator circuit of the extracorporeal circulation apparatus 10 may be determined, displayed, and controlled.

Further, the extracorporeal circulation condition of the extracorporeal circulation apparatus 10 may be automatically changed on the basis of the prediction, and an administration system for reducing the onset risk of the postoperative complication may be controlled on the basis of the prediction.

A specific example is as follows.

When information indicating a decrease in BP (blood pressure) as vital information is obtained during the operation, a prediction that a "cerebral infarction risk" is increased when the BP (blood pressure) is the same after two minutes is made by the learned algorithm on the basis of a set value, a specified value, or the like, the circulation condition of the extracorporeal circulation apparatus 10 is changed, and a "start of vasopressor administration" or the like is performed.

At this time, a step of confirming an intention of the anesthesiologist may be added. In this case, vasopressor administration is actually started when a displayed instruction is accepted.

In addition, as another example, when information indicating a decrease in Ht (hematocrit value) is obtained during the operation, a prediction that "an AKI risk is decreased when water content is reduced" is made by the learned algorithm, the circulation condition of the extracorporeal circulation apparatus is changed, and "opening of a hemoconcentrator circuit," "preparation of a diuretic," or the like is performed.

Thus, according to the present modification, automatic operation of the heart-lung machine as the extracorporeal circulation apparatus 10 that performs parameter control with even the onset risk of a postoperative complication taken into consideration can be performed.

In addition, while the present embodiment is configured to display predictive information or operate the extracorporeal circulation apparatus 10 according to the predictive information, the present invention is not limited to this, but includes a medical action of optimizing the circulation condition of the extracorporeal circulation apparatus 10 according to the predictive information, the medical action being performed by the doctor.

### (Second Embodiment)

Much of a configuration according to a present embodiment is similar to that of the foregoing first embodiment, and therefore description of an identical configuration will be omitted. In the following, description will be made mainly of differences.

Unlike the foregoing first embodiment, the present embodiment is configured to predict an "index related to medical economy" using the step of the "learned algorithm."

Specifically, a prediction of the number of ICU stay days, a prediction of a death probability, and the like are performed by the "learned algorithm" on the basis of the "preoperative data" and the "intraoperative data" described above or the like, and these predictions are stored as an "index related to medical economy."

In addition, the embodiment is configured to predict whether a medical action related to "cost" such as an extension of the number of ICU stay days or CRRT (continuous renal replacement therapy) will occur in the future by using the step of the "learned algorithm" on the basis of the "preoperative data" and the "intraoperative data" described above or the like.

FIG. 4 is an example of calculating a "medical cost" as an example of medical economy. FIG. 5 is a diagram illustrating data as a basis of the medical cost.

The present embodiment generates a "two-dimensional prediction of a position of a risk on a matrix table" combining information regarding the "index related to medical economy" or a "prediction of a possibility that a medical action related to cost will occur in the future" in FIG. 4 or the like with the "prediction of the onset risk of a postoperative complication" in the foregoing first embodiment, and displays the two-dimensional prediction on the display section 46.

A medical worker such as a doctor can thereby recognize these relations at a glance.

That is, the matrix table can two-dimensionally display a group of high onset risks of a main complication and a group of low onset risks of the main complication, and a group of high probabilities of the number of ICU stay days, in-hospital death, CRRT introduction related to medical economy and a group of low probabilities of the number of ICU stay days, in-hospital death, CRRT introduction related to medical economy, and predict and display a region to which a transition is made in the matrix table during an ICU stay.

Here, the high groups and low groups are divided as follows, for example.

For example, the group of high onset risks of the complication and the group of low onset risks of the complication are divided according to the presence or absence of an onset of the complication within seven days or the like, and the number of ICU stay days is divided according to whether or not a stay in the ICU for three days or more is made, for example.

In addition, the in-hospital death is divided according to whether or not the in-hospital death is a death within seven days.

FIG. 6 illustrates a "matrix table" of an "AKI onset risk" as an example of the onset risk of a postoperative complication and a "medical cost (medical economy or the like)" as an example of medical economy or the like. FIG. 7 illustrates a "matrix table" of the "AKI onset risk" and an "edema."

As illustrated in FIG. 6 and FIG. 7, by using such matrix tables, it is possible to two-dimensionally display a group of high onset risks of a main complication and a group of low onset risks of the main complication, and a group of high risks of occurrence of a symptom that needs an action such as an edema or an infection and a group of low risks of occurrence of the symptom, and predict and display a region to which a transition is made.

In addition, in the case of FIG. 6, when the AKI risk is "high," and the medical cost is "high," divisions can also be further made and displayed according to costs incurred for three and seven days from hospitalization or the like.

In addition, while the present embodiment displays the two-dimensional matrix tables of four events, the present invention is not limited to this, but when in-hospital death is taken on an axis of abscissa, for example, 2 × 3 events including survival and deaths within 7 days and 30 days, and the like can be adopted. The present invention is thus not limited to four events.

As described above, according to the foregoing embodiment, high risk patients can be stratified in advance by using data obtained during the operation, and a cardiac surgery or an extracorporeal circulation procedure with attention paid to a prognosis indicating that an AKI or the like is less likely to be caused is made possible. Thus, even when the doctor or the like performs the same procedure, a useful determination can be made from patient QOL and medical economy.

In the foregoing embodiment, the learned algorithm used for prediction is constructed from data obtained in advance. In addition, the data obtained in advance includes data obtained from electronic medical records or the like recorded in the EHR server 50 or the like.

When the learned algorithm is constructed, the learned algorithm is prepared by a method such as regression analysis, machine learning, or a neural network (NN) using preoperative data and intraoperative data in the past.

The preoperative data includes, for example, the above-described operative method and the like. The intraoperative data includes, for example, the above-described vital information and the like. An algorithm construction can be performed by using the data associated with data on the postoperative complication.

The construction of the algorithm may use not only ordinary linear regression analysis, logistic regression analysis, SVM (Support Vector Machine), a random forest, a decision tree, and the like, but also ensemble learning using a plurality of models.

For example, LightGBM, Gradient Boosting, and the like correspond to this.

These methods are constantly updated to highly accurate methods, and therefore a method providing more accuracy may be selected.

In a neural network, it suffices to use a method such as a Conventional Neural Network (CNN), a Recurrent Neural Network (RNN), or Generative Adversarial Networks (GAN) according to a kind of data and the format of data obtained.

The learned algorithm may be not only constructed from data obtained in advance, but also configured to record data obtained in real time in an electronic medical record or the like and thereby update data in the electronic medical record or the like.

In that case, a prediction can be performed while original data necessary for algorithm construction is updated in an algorithm generating section cooperating with the data obtaining section 41, and an update is performed in conformity with a present situation and in real time.

As for an index of accuracy in that case, a confusion matrix (Confusion Matrix) that makes an evaluation on the basis of sensitivity and specificity is performed, AUROC (Area Under Receiver Operating Curve) may be used, and an accuracy rate (Accuracy), precision (Precision), reproducibility (Recall), an F-value (F1 score), and the like are used.

This may be automatically performed within a "prediction system," or a selection may be allowed to be made as to which model to use each time the original data is updated.

In addition, because the accuracy of these models greatly affects a treatment result of the patient and a prognosis, there may be a configuration that performs external control via communication in addition to a selection within the hospital or the like.

Embodiments of the present invention have been described above. However, the present invention is not limited to the foregoing embodiments, but can be modified variously without departing from claims.

### Description of Reference Symbols

- 2:: Blood sending pump
- 3:: Reservoir
- 4:: Oxygenator
- 5:: Blood removing cannula
- 6:: Blood sending cannula
- 7:: Suction cannula
- 8:: Vent cannula
- 9:: Cardioplegia cannula
- 10:: Extracorporeal circulation apparatus
- 11:: Second connecting tube
- 12:: Heat exchanger
- 13:: Solution feeding tube
- 14:: Artery filter
- 15:: Air bubble detector
- 16:: Suction cannula tube
- 17:: Suction pump
- 18:: Vent cannula tube
- 19:: Vent pump
- 20:: Cardioplegic solution bag
- 21:: Cardioplegic solution tube
- 22:: Cardioplegic solution pump
- 23:: Heat exchanger
- 24:: Blood removing tube
- 30:: First connecting tube
- 40:: Controller
- 41:: Data obtaining section
- 42:: Control section
- 43:: Integrated data recording section
- 44:: Data input section
- 45:: Data recording section
- 46:: Display section
- 50:: EHR server
- 51:: Cloud
- 53:: External communication and display medium
- 60:: Vital data generating device
- 61:: Patient data generating section
- 62:: Patient data analyzing section
- 63:: Edge side data display section

## Claims

1. A prediction display system, wherein
an onset risk of a complication occurring after an operation is predicted using at least one of pH (hydrogen ion exponent), pO2 (oxygen partial pressure), Ht (hematocrit value), Hb (hemoglobin), DO2 (transportation amount of oxygen), and pCO2 (arterial blood carbon dioxide partial pressure) as parameters of blood gas taken before the operation and during the operation, and the onset risk of the complication occurring after the operation is displayed.

2. The prediction display system according to claim 1, wherein
the onset risk of the complication after the operation is predicted using at least one of pH, pO2, Ht, Hb, DO2, and pCO2 as the parameters of the blood gas in data obtained during operation of an extracorporeal circulation apparatus and at least one of a pump flow rate, a pump rotational speed, a temperature, a blood circulation time, and an aorta interruption time as operating conditions of the extracorporeal circulation apparatus, and the onset risk of the complication after the operation is displayed.

3. The prediction display system according to claim 1 or 2, wherein
the complication is an acute kidney injury occurring after the operation.

4. The prediction display system according to claim 3, wherein
the onset risk of the acute kidney injury after the operation is predicted using at least one of an arterial pressure, a heart rate, an ECG (electrocardiogram), a urinary volume, a peripheral oxygen saturation, and a deep body temperature as a temperature within a body as vital data, and the onset risk of the acute kidney injury after the operation is displayed.

5. The prediction display system according to claim 1 or 2, wherein
the onset risk of the complication is an onset risk of at least one of a low cardiac output syndrome (LOS), an acute kidney injury (AKI), a cerebral infarction, a mediastinitis, and an acute respiratory distress syndrome (ARDS) as the complication after the operation.

6. The prediction display system according to claim 5, wherein
a prediction is performed using the onset risk, and an extracorporeal circulation condition of the extracorporeal circulation apparatus, the extracorporeal circulation condition being likely to reduce the onset risk of the complication after the operation, is analyzed and displayed.

7. The prediction display system according to claim 6, wherein
a change in flow rate and rotational speed of either a blood removing pump or a blood sending pump of the extracorporeal circulation apparatus is suggested.

8. The prediction display system according to claim 6, wherein
whether or not there is a need for a hemoconcentrator circuit of the extracorporeal circulation apparatus is determined and displayed on a basis of the onset risk of the complication after the operation.

9. The prediction display system according to claim 6 or 7, wherein
the extracorporeal circulation condition is automatically changed on a basis of the prediction.

10. The prediction display system according to claim 5, wherein
an administration system for reducing the onset risk of the complication after the operation is controlled on a basis of the prediction.

11. The prediction display system according to any one of claims 1 to 10, wherein
the onset risk of the complication is predicted using at least one of an age, a sex, an address, a race, a family structure, an anamnesis, and a preoperative test value that affect a prognosis as characteristics of a patient, and the onset risk of the complication is displayed.

12. The prediction display system according to any one of claims 1 to 11, wherein
the onset risk of the complication is predicted using at least one of an operative method, a lesion site, an operation time, an operator, an anesthesiologist, a dosage, a kind of medicine, an anesthesia induction time, and presence or absence of a blood transfusion as information regarding the operation, and the onset risk of the complication is displayed.

13. A prediction display system, wherein
an index of at least one of the number of ICU (intensive care unit) stay days, presence or absence of in-hospital death, and presence or absence of continuous renal replacement therapy (CRRT) introduction related to medical economy is predicted using at least one of pH, pO2, Ht, Hb, DO2, and pCO2 as parameters of blood gas in data obtained during operation of an extracorporeal circulation apparatus and at least one of a pump flow rate, a pump rotational speed, a temperature, a blood circulation time, and an aorta interruption time as operating conditions of the extracorporeal circulation apparatus, and the index is displayed.

14. The prediction display system according to claim 13, wherein
an onset risk of a main complication and another management item are taken on an axis of ordinate and an axis of abscissa and are two-dimensionally displayed on a matrix table, and a region to which a transition is made in the matrix table during an ICU stay is predicted and displayed.

15. The prediction display system according to claim 14, wherein
the matrix table two-dimensionally displays a group of high onset risks of the main complication and a group of low onset risks of the main complication, and a group of high probabilities of the number of ICU stay days, in-hospital death, and CRRT introduction related to medical economy and a group of low probabilities of the number of ICU stay days, in-hospital death, and CRRT introduction related to medical economy, and the region to which a transition is made in the matrix table during the ICU stay is predicted and displayed.

16. The prediction display system according to claim 14, wherein
the matrix table has a group of high onset risks of the main complication and a group of low onset risks of the main complication, and a group of high risks of occurrence of a symptom that needs an action such as an edema or an infection and a group of low risks of occurrence of the symptom, and the region to which a transition is made two-dimensionally in the matrix table is predicted and displayed.

17. A treatment method comprising:
predicting an onset risk of a complication using at least one of pH, pO2, Ht, Hb, DO2, and pCO2 as parameters of blood gas in data obtained during operation of an extracorporeal circulation apparatus and at least one of a pump flow rate, a pump rotational speed, a temperature, a blood circulation time, and an aorta interruption time as operating conditions of the extracorporeal circulation apparatus;
displaying the predicted onset risk of the complication on a screen; and
optimizing a circulation condition of the extracorporeal circulation apparatus on a basis of predictive information on the onset risk of the complication, the predictive information being displayed on the screen.
